# EUROPEAN PATENT SPECIFICATION

(11) **EP 1 804 698 B1**
(45) Date of publication and mention of the grant of the patent: **04.07.2012**
(21) Application number: 04794049.9
(22) Date of filing: 04.10.2004
(51) Int. Cl.: A61B 17/58

(54) **INTRAMEDULLARY NAIL DEVICE FOR REPAIRING LONG BONE**
MARKNAGEL ZUR REPARATUR VON RÖHRENKNOCHEN
DISPOSITIF D'ENCLOUAGE CENTROMEDULLAIRE DE REPARATION D'UN OS LONG

(43) Date of publication of application: 11.07.2007
(73) Proprietor: Saint Louis University, St. Louis, MO 63104 (US)
(72) Inventor: BLEDSOE, John Gary, Saint Louis, Missouri 63119 (US); MOED, Berton Roy, Saint Louis, MO 63105 (US); CONDOOR, Sridhar, Fenton, MO 63026 (US); SHIELDS, Kevin Anthony, Champagne, IL 61820 (US); ALFORD, Timothy Patrick, Fenton, MO 63026 (US); MILLER, Robert J IV, Parma, OH 44134 (US)
(74) Representative: Pilkington, Stephanie Joan
(86) International application number: PCT/US2004/032546
(87) International publication number: WO 2006/041460

(56) References cited:
- US-A- 5 415 660
- US-A- 6 127 597
- US-A1- 2002 177 866
- US-A1- 2003 181 918
- US-B1- 6 783 530

## Description

### BACKGROUND OF THE INVENTION

### Field of the invention

The invention is directed to devices to repair bone tissue, more particularly to an intramedullary nail and its surgical placement to repair a humerus.

### Description of the related art

### Intramedullary nails

Intramedullary nails have been used to repair long bones since the 1930s, with the introduction of the Smith-Peterson nail to repair femur fractures. Since that time, improvement and variations of the intramedullary nail have been made. Screws have been introduced to fix the nails in place and to prevent rotation. Newer devices have been developed that comprise expansion devices such as wedges, deploying arms, anchor blades and tangs, which help fix the nail in place and to prevent rotation within the intramedullary canal. A discussion of the intramedullary nail art related to the present invention is discussed in U.S. Patent No. 6,488,684. Regardless of the design of the nail or method of fixing the nail within the intramedullary canal, all intramedullary nails to date are placed into the intramedullary canal through the proximal or distal end of the long bone coaxial to the cross section of the canal, that is directly inline with the axis of the canal.

US 6,127,597 discloses a self-expending intramedullar fixture constructed of two sheets of resilient biocompatible material rolled together tightly in cylindrical form.

US 6,783,530 discloses an expandable orthopedic device comprising a plurality of resilient spine elements longitudinally arranged.

US 2002/177866 discloses an inflatable device for use in restoring the anatomy of diseased or fractured bone.

US 2003/181918 discloses a device for aligning and reducing sections of fractured bone.

### Medical Grade Alloys

Intramedullary nails must be made of biocompatible materials. The most commonly used materials for intramedullary nails are stainless steel and titanium. Biocompatibility is the foremost concern when implanting any foreign object into the human body. The human body will actively attempt to destroy any unknown material that enters the body as a defense mechanism, known as the foreign body response. The biocompatibility of a material is closely based on the reactions between the surface of the material and inflammatory host response. Although ally materials implanted into the body cause an inflammatory host response, certain materials, such as titanium, produce less of a response and are considered to be biologically inert. The more biologically inert a material is, the safer it is to implant in the body.

The body's response to a metal implant is different in bone than in soft tissue. Within two to three days after implantation, stem cells from the bone develop into osteoblasts, which along with fibroblasts form a layer near the implant surface. A collagen rich extra cellular matrix forms, which is followed by mineralization. In more vascularized tissue, the implant will be covered by a blood clot including leukocytes, thrombocytes, and various proteins. Inflammatory cells, such as monocytes and macrophages, arrive at the implantation site to remove debris and foreign materials. This foreign body response can begin to degrade the material causing even more inflammation and thrombosis. The implantation site will become painful and swollen as the body rejects the implant. Materials such as titanium and stainless steel are used in medical devices and implants because they are relatively biologically inert and do not induce a significant foreign body response.

A newer nickel titanium alloy called Nitinol is increasingly being used to repair damaged tissue. The unique properties of nickel titanium alloy were discovered in 1961 in a Navy lab. The common name, Nitinol, stands for Ni-Ti Naval Ordnance Laboratory. Nitinol is a shape memory metal, which undergoes martensite-austenite transition as a result of a change in temperature. This property of Nitinol has shown its usefulness in internal fixation of bone fractures, endoprostheses, spine surgery, cranial facial surgery and oral maxillofacial surgery (referenced in Youyi, Chu. Orthopedic Applications of NiTi Shape Memory Alloys in Chine. SMST-2000 Conference proceedings. 2001), as well as in animal experiments on bone deformation (Kujala et al., Bone modeling controlled by a nickel-titanium shape memory alloy intramedullary nail, Biomaterials 23 (2002) 2535-2543; and Kujala et al., Comparison of the bone modeling effects caused by curved and straight ruckel-titanium intramedullary nails, Journal of Materials Science: Materials in Medicine 13 (2002) 1157-1161.

Materials tested for use in the body are graded on the host's reaction induced by the material and the degradation of the material in the body environment. The largest concern in using Nitinol is the large concentration of nickel within the material. A small percentage of the population is allergic to nickel. In high doses, nickel is toxic to all individuals. However, the nickel molecules in the material are chemically bonded to the titanium molecules and do not leach out into the body in high doses. Most observations from extracted Nitinol implants show little or no corrosion with the highest corrosion rate being 0.46 mm/year (referenced in Youyi, Chu. Orthopedic Applications of NiTi Shape Memory Alloys in Chine. SMST-2000 Conference Proceedings. 2001). These tests conclude that the nickel in Nitinol does not pose a risk to the patient

In addition, several coatings have been created to further stabilize the material surface to ensure there is no corrosion or nickel bleaching. One coating, calcium phosphate, has been used to create a more physiologically stable surface, which could help prevent nickel release. The layer (5-20 µm) is applied by dipping the implant in the calcium phosphate solution. The calcium phosphate creates a physiological surface to which leukocytes and platelets adhere more readily. This layer has been proven to decrease the foreign body response and prevent nickel release that may occur slightly within the first few days of implantation and during high load bearing situations (referenced in Choi et al., "Calcium phosphate coatings of nickel-titanium shape-memory alloys. Coating procedure and adherence of leukocytes and platelets", Science Direct, 2003). However, in vivo tests of Nitinol, described in Choi et al., 2003, demonstrated that Nitinol performs similarly to stainless steel, even without any surface treatment (see also Shabalovskaya, S. A., "Surface, corrosion and biocompatibility aspects of Nitinol as an implant material," Nitinol Sciences, Consulting, 2001)

### Current Surgical Procedure

Due to the complex musculoskeletal anatomy of the shoulder and elbow, the repair of a fractured humerus bone is a complicated medical procedure. The current procedure is time consuming, invasive, and often accompanied by post surgical complications. First, an incision is made through the skin at the shoulder, exposing the rotator cuff. The rotator cuff is severed to expose the proximal portion of the humerus. A medical drill is used to cut through the ossified outer layer of the bone. A guide wire is dropped into the humerus bone canal to provide a path for the metal rod to follow as it crosses the fracture. A reamer is used to extract the marrow and enlarge the canal. An intramedullary nail, which is usually made of a titanium alloy, is hammered into the reamed out section of the bone following the path of the guide wire. Once in place, surgical screws are inserted through the bone into predrilled holes in the nail to hold the proximal and distal ends of the nail in place. The rotator cuff and initial incision through the skin are then sutured.

Although the procedure repairs the fractured bone, it often causes damage to the rotator cuff, possibly requiring additional surgery. Also, if the rod needs to be removed because of infection or other complications, the rotator cuff must be severed once again, causing even more damage. Thus, there is a great need for new and improved devices and surgical methods (not claimed) to repair a fractured humerus, which reduces surgical complications.

### SUMMARY OF THE INVENTION

Provided herein is an intramedullary nail that can transition from a flexible state to a non-flexible state. The nail comprises a shape memory metal, which is preferably a nickel-titanium alloy, having a martensite (bendable) phase at a physiologically safe temperature and an austenite (stiff) phase at a physiological temperature. In the martensite state, the nail is bent off its cylindrical axis to accommodate insertion of the nail into an intramedullary canal. In the austenite state, the nail assumes its therapeutically effective shape and becomes stiff. The nail may comprise a single cylinder of shape memory metal, or a combination of multiple cylinders of shape memory metal. The nail may be made of any biocompatible material, such as stainless steel, titanium or the like, or other material coated with a biocompatible coating.

The flexible nail comprises a shape memory metal having a martensite state at a physiologically safe temperature (e.g., ≥0°C) and an austenite state at physiological temperature (e.g., ~37°C). In a preferred embodiment, the shape memory metal is a nickel-titanium alloy. In this embodiment, the nail is cooled to the martensite state (i.e., a martensite temperature) and bent to a shape to accommodate the insertion of the nail into an incision that is not aligned in-line to the intramedullary canal (off-center incision). The nail is then Inserted through the off-center incision and into the intramedullary canal using a jig cooled to a martensite temperature. After the nail has been placed into the intramedullary canal, the rail warms to an austenite temperature, taking on its therapeutically effective shape and becoming stiff. The nail may be secured using any means, such as barbs, tabs, wedges, screws and the like, as is known in the art.

### BRIEF DESCRIPTION OF THE DRAWINGS

Figure 1 depicts the stress / strain curve of a Nitinol rod in austenite (high temp) (A) and martensite (low temp) (B) phases.

Figure 2 depicts a CAD drawing of a link of a link and sleeve nail system.

Figure 3 depicts a line drawing of a single link, further showing the flexible (A) and rigid (B) link positions. Female end (C), Male end (D) and pins (E) are also depicted.

Figure 4 depicts a simple drawing of an intramedullary nail, most useful for repair of a fractured humerus. Longitudinal and cross sections are depicted.

Figure 5 depicts a rendition of a humerus and a Nitinol nail spanning a fracture.

Figure 6 depicts a diagram showing the deflection vector for the humerus and Nitinol nail used to determine forces.

Figure 7 depicts a cross section map of a Nitinol nail in a bone, to determine heat transfer parameters.

Figure 8 depicts a time vs. temperature equilibration curve for Nitinol nail.

Figure 9 depicts an improved nail placement jig, having a hollow (annular) tube (A) to accommodate chilled saline. Hammer (B) and locking screw centering device (C) are depicted in Figure 9.

Figure 10 depicts a time vs. temperature equilibration curve for Nitinol nail in chilled saline.

Figure 11 depicts a time vs. temperature equilibration curve for Nitinol nail in chilled saline.

Figure 12 depicts a line drawing of an intramedullary nail showing suggested screw locations (A) and distal taper. Proximal end (B) and distal end (tapered) (C) are depicted in Figure 12.

Figure 13 depicts an unexploded (A) and an exploded (B) views of a nail insertion system. Jig with hollow core (X), Nitinol intramedullary nail (Y) and guidewire (Z) are depicted.

Figure 14 depicts a cross section (A) and a longitudinal section (B) of a section of a braided Nitinol nail.

### DETAILED DESCRIPTION OF SEVERAL EMBODIMENTS

Applicants have invented an intramedullary nail device that will provide support and alignment for any fractured long bone (especially a humerus) during the healing process. Along with providing this alignment and structure, the subject intramedullary nail device can transition between a flexible state and a rigid state, which allows for greater flexibility in selecting an incision site for insertion of the nail into an intramedullary canal. This especially opens the possibility of repairing the humerus without damaging the rotator cuff and surrounding tissue. The intramedullary nail device also enables a surgical procedure that would be less invasive allowing for the healing time of the patient to be reduced. The current intramedullary nail insertion procedure followed in the art takes approximately three hours to perform. The subject intramedullary nail device which can transition from flexible to stiff, could potentially drastically reduce this time to under an hour.

While the subject intramedullary nail device can be used in the repair of any bone and is not intended to be limited to any particular bone, it is especially useful in the repair off fractures in the humerus, given the complex anatomy of the elbow and shoulder. Due to the fact that the new surgical procedure and intramedullary nail device of the instant invention prevents the damage of the rotator cuff, the chance of additional surgery is greatly reduced. This alone can help the patient save money and pain from an additional procedure. Furthermore, the time needed for rehabilitation is expected to be drastically decreased, resulting in additional cost savings to the patient, the community and third party payers. The new intramedullary nail device of the instant invention can help the surgeon and hospital by reducing the manpower and time required per surgery.

### Example 1: Link and sleeve nail system

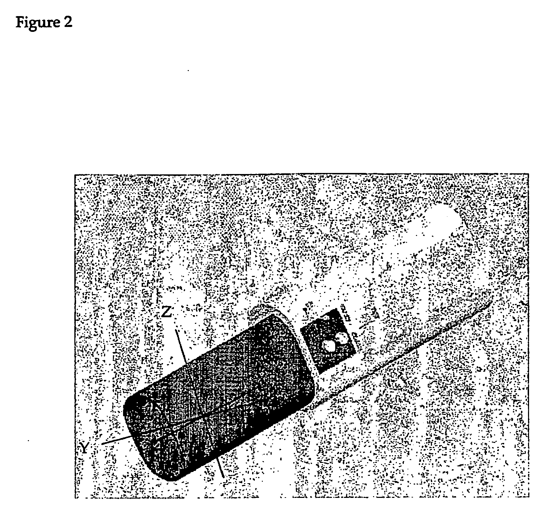

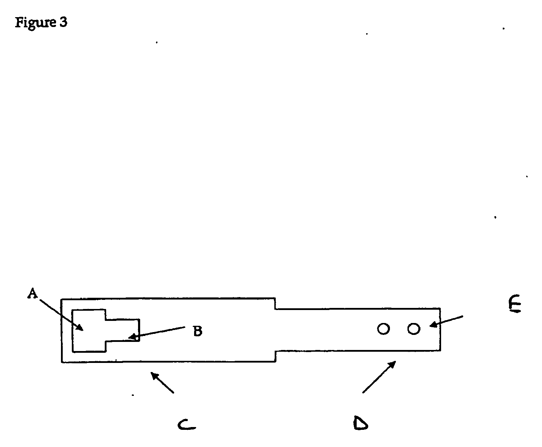

An intramedullary nail device, which is graphically described in figures 2 and 3, is based on a simple chain link system The entire system may comprise any number of medically approved materials suitable for implantation, such as for example medical titanium, a metal that has already been used in FDA approved implantation devices. The interface between two links is shown in Figure 2 Batch link will be hallow containing both a male and female end (shown in Figure 3). The male end will fit flush inside the female end on the Y-axis. There will be space between links on the Z-axis to allow movement. This movement will be enough to allow for the links to be inserted through the incision and into the hole in the bone. A piece of wire or other flexible material will be attached to the distal link, threaded through each link and connected to a screw head at the proximal link. When there is no tension in the wire, the pins of the male end will be at position "A" in Figure 3. When the screw is turned, the wire threaded through the chain links will become tighter, bringing the links together. This will move the pins from "A" to "B" (Figure 3). When the links are tight relative to each other, relative movement of each rod is prevented, creating a stiff rod. According to this particular example, the ends of the apparatus will be very similar to current nails in the art that are being used in humerus fracture repair in that both ends of the apparatus have predrilled holes that will allow screws to be inserted in order to fully secure the rod after it has been placed in the bone.

This particular device described in this example will allow the surgeon to decide when he or she would like to stiffen the rod after it has been inserted into the intramedullary cavity, therefore removing any time constraint imposed by a martensite-austenite transition event. Any removal of a time constraint makes for a surgeon friendly device.

In some cases, removal of an intramedullary nail is necessary. In this particular example, the linkage system design allows for easy removal of the nail. By simply loosening the screw and wire, and thus relieving any tension in the system, the links unlock, allowing the links to rotate relative to each other and making the entire device flexible once again.

In some situations, some pins may shear at removal, thus causing some links to separate and remain inside the bone. Thus, in one aspect of this embodiment, a plastic polymer sleeve can surround the device, trapping and links that may separate during removal. The sleeve also makes the device smoother, allowing for easier insertion and removal.

It is envisioned that gaps in the links may potentially allow bone to grow into the system of links, thus potentially making removal of the rod difficult. One of many ways to solve that problem is by using a plastic polymer sleeve as described above (supra). The sleeve would be tough enough to prevent bone growth between gaps, but soft enough to allow movement when inserting the device into the body. The sleeve may be fixed to the bottom end of the nail, allowing the links to move freely within the sleeve.

Given that patients vary in long bone length and canal diameter, this particular device can be fitted for various sized patients. Individual links could be added or removed to accommodate the size of the patient.

### Example 2: Shape memory intramedullary nail

### General Properties of Body Temperature Nitinol

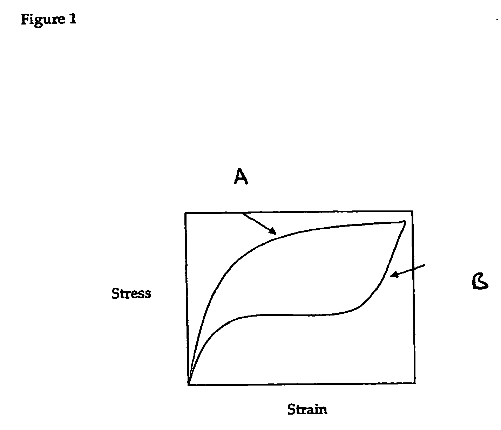

The shape transformation of a material, such as for example a nickel-titanium alloy (e.g., Nitinol), occurs between the austenite and martensite phases. By varying the amount of nickel and titanium along with small amount of other metals, the ' transformation temperature range of Nitinol can be altered. An exemplary and non-limiting Nitinol alloy that is useable at physiologically safe and useful temperature range, i.e., "body temperature Nitinol", has an austenite start temperature of 15°C and an austenite finish temperature of 35°C. To obtain its memory capability, a set process should be followed. The material is set in a mold and baked at around 500°C for about an hour, then quenched. Following the quenching process, the metal can be deformed by tension/compression, bending or torsion, and will return to its original, set shape when heated. Figure 1 shows the basic stress strain diagram defining how the material behaves at various temperatures.

Figure 1 shows that Young's modulus is greater at a high temperature phase than at a low temperature phase. This is based on the simple equation Young's Modulus = Stress/Strain.

The body temperature Nitinol of this particular non-limiting embodiment contains about 55.5 % nickel, 0.05% carbon, 0.005% hydrogen, 0.05% oxygen, 0.05% copper and the remaining, titanium. It also posses two different Young's Moduli: 82.74 x 10⁹ Pa (12x10⁶ psi) in its austenite phase and 41.37x10⁹ Pa (6x10⁶ psi) in the martensite phase (see Buehler, W. J. and R. C. Wiley, "Nickel-Base Alloys", U.S. Pat. No. 3,174,851, March 23, 1965.

### Brief Description of Single Piece Nail

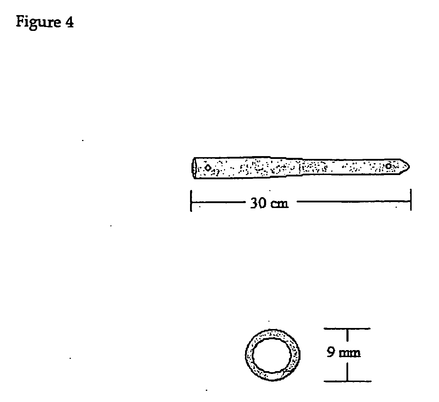

The image depicted in Figure 4 shows the basic dimensions and views of a single intramedullary nail device comprising a shape memory metal, wherein the austenite state is at physiological temperatures (e.g., 37°C) and the martensite state is between 0° and 15°C. The length of the device would be to accommodate the intramedullary canal of a bone, e.g., about 254 ± 50 mm in length, with a diameter tapering off from about e.g. 10 mm to e.g. 9 mm from proximal to distal end respectively (see an exemplary device depicted in Figure 4.) The device of this particular aspect would have predrilled holes for self-tapping screws that are approximately 4 mm wide. These screws would allow for fixation of the humeral shaft fracture during the healing process.

The device could be cooled below 15° C to its malleable martensite form in order to set it to its desired shape for insertion. After the hole is drilled into the bone and the cavity is reamed, the rod would be slowly inserted into the humerus. As the device warms to 37°C, the Nitinol nail would transition into the originally manufactured shape, which would conform to the straight intramedullary canal cavity. Once the rod is in the bone the proximal and distal screws would be inserted to fix the nail to the long bone shaft.

This particular exemplary nail is made with the same or very similar dimensions to a standard titanium nail for a particular bone (e.g., humerus), but is made instead with body temperature Nitinol, allowing for insertion points away from the rotator cuff for humerus application. The device has certain mechanical properties to properly fix the humeral fracture and allow for proper healing. During the healing process, the rod must be able to withstand the stresses caused by forces on bone.
Nitinol has very similar mechanical properties to medical titanium, which has been proven to have adequate stiffness to satisfy the conditions needed for proper healing of a bone. Using a 1.27 cms (0.5-inch) deflection test, the particular Nitinol rod compares very closely with a titanium rod. When the Nitinol was warmed up to body temperature (austenite phase) it required 369.2N (83 pounds to force) to deflect the rod 1.27cms (0.5 inches), while the titanium rod with identical dimensions require 387N (87 pounds). From this test one skilled in the art would reasonably expect that the Nitinol rod, while in the austenite phase, would satisfy the structural demands of fixing and supporting a fractured humeral bone.

### Single piece Nitinol nail model

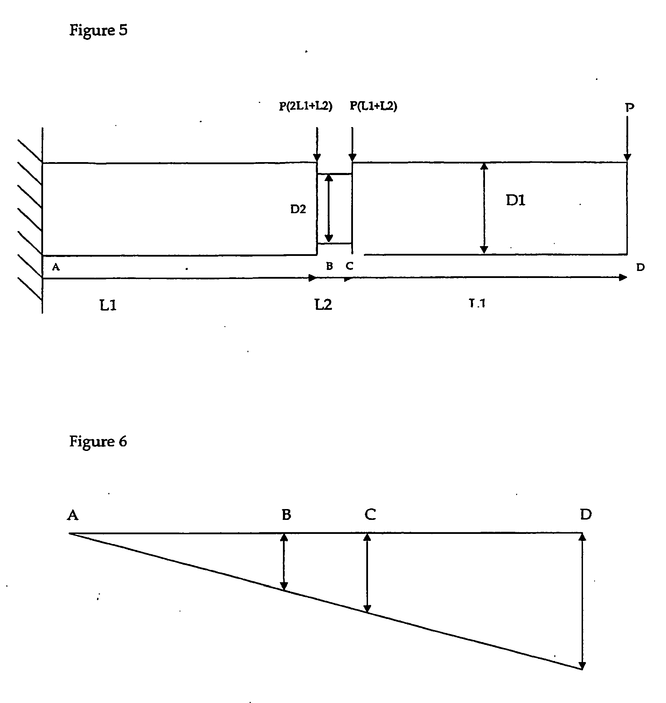

Figure 5 depicts the device in a bone. Position A, at the left side of the diagram, represents the proximal or shoulder end of a humerus. Position D, at the right side of the diagram, represents the distal or elbow end of a humerus. AB depicts the area from the shoulder to the fracture, BC depicts the area of the rod crossing the fracture and CD depicts the area from the fracture to the elbow. To model the bending forces of the instant exemplary Nitinol nail for use in a humerus, it was assumed that the proximal end is fixed in all directions and rotations and the load was applied to the elbow (distal) end (see Figure 6).

Equations were developed to calculate the deflection from AB, BC and CD. The total deflection was then found based on these three calculations. Only the fracture, which is a very small portion, was subject to bending. The length of the fracture was modeled to be very small as the rod compresses the bone together to close the fracture tightly. At every other section away from the fracture, the bone was taking most of the load. Calculations were made according to Gere, James, Mechanics of Materials, Brooks/Cole, 2001, pg. 646, examples 9-10. The following values were used in those calculations: D1 (bone diameter) = 2.2 cm (0.866 inch); D2 (outer diameter of nail) = 0.9 cm (0.354 inch); D2i (inner diameter of nail) = 0.4 cm (0.157 in); L1 = 12.67cm (4.99 inch); L2 = 0.00 cm (Figure 5). Accordingly, a force of 347N (78 Ib) issued at the elbow deflects the Nitinol rod 1.27 cm (0.5 inch) when in austenite form. This is comparable to a titanium rod, which requires 355.8N (80 Ib) for the same deflection.

Another calculation was performed to determine the force needed to overcome the yield strength of Nitinol in martensite form. Accordingly, the yield strength of the Nitinol in martensite form was calculated to be 137.9 MPa (20 ksi). The device is modeled as a cantilever beam supported at the top. It was determined that 40N (nine (9) pounds of force) would need to be applied, which would yield a stress of about 144.8 MPa (21 ksi) to overcome the yield strength of the martensite Nitinol to bend it. 40N (nine (9) pounds of force) can easily be applied by the surgeon to shape the rod before surgery.

A calculation was performed to determine the force needed to break the bone during nail insertion. It was determined that a force of 542.7N (122 Ib) is needed to break the bone during insertion. This is far more than what would be applied to insert a martensite nail, making the procedure safe.

The force needed to buckle the Nitinol nail of this example was calculated to be around 1.78 kN (400 pounds) which is far more than would be applied during the insertion process, making this device safe from buckling.

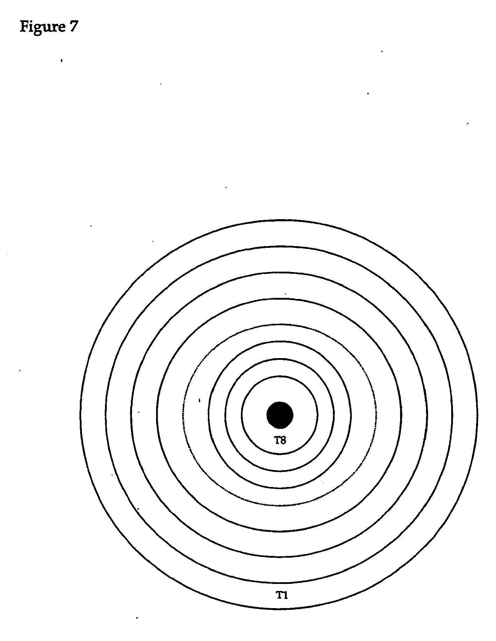

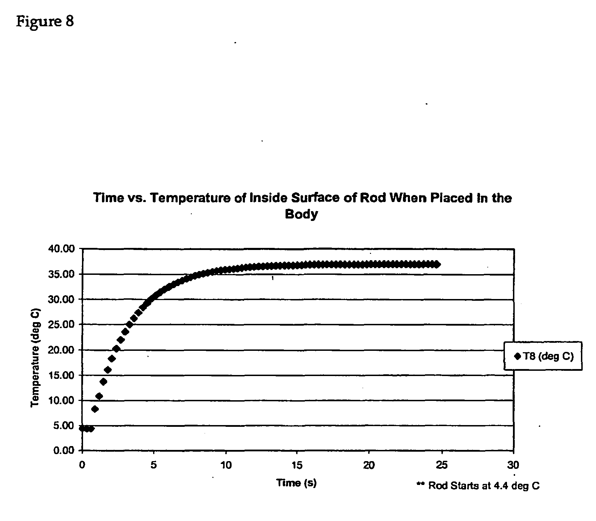

A heat transfer calculation was performed on the Nitinol nail of this example. Figure 7 depicts the cross section of a nail and a bone. The dotted line in that figure represents the interface between bone and rod. The heat transfer calculation that was performed using a marching solution. The equations considered a conduction boundary layer between bone and rod and bone and body. Accordingly, it was determined that the nail of this example inserted into the body would heat from 4.4°C to 37°C (the austenite finishing temperature, thus yielding a stiff rod) in 25.6 seconds (Figure 8).

### Improved nail placement jig

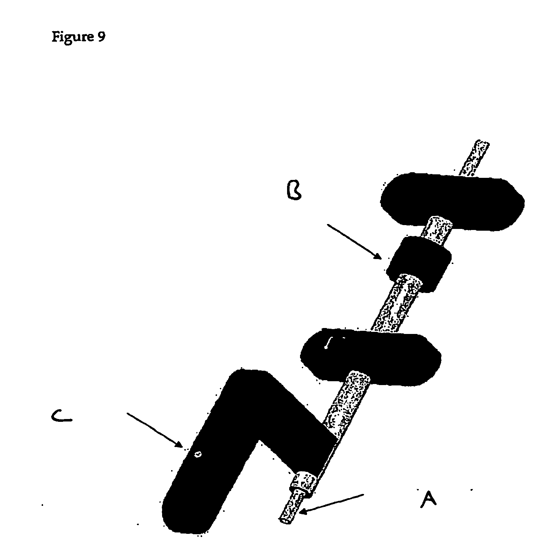

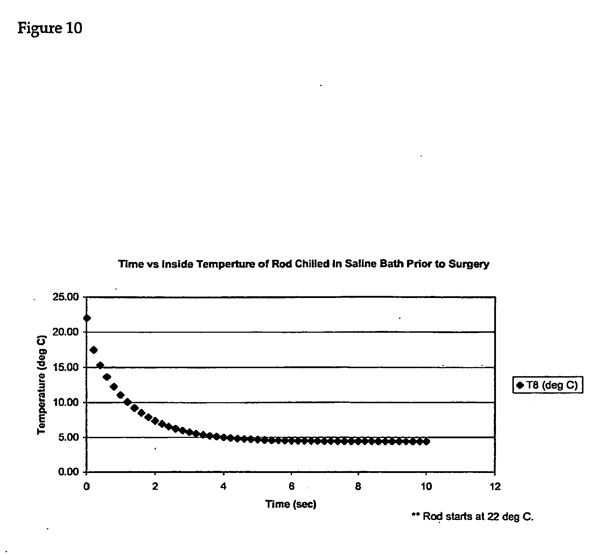

In order to place less of a time constraint on the surgeon, also provided is an improved nail insertion device (a.k.a. improved jig), which allows the intramedullary nail to remain at a temperature below the austenite start temperature while in the jig (Figure 9). The improved jig resembles an insertion device that is currently in use. However, on the improved jig, the solid rod has been drilled out to form an annulus. The purpose of this annulus is to provide a path to inject chilled saline into the Nitinol nail during the insertion procedure. The improved jig is fastened onto the Nitinol nail to form an assembly. The assembly is then placed back into a chilled saline bath in order for the rod to reach its martensite phase (infra). Based on a heat transfer calculation considering a convection boundary inside the rod, it would take approximately 10 seconds for the rod to reach its martensite temperature when completely submerged in the chilled saline bath Figure 10).

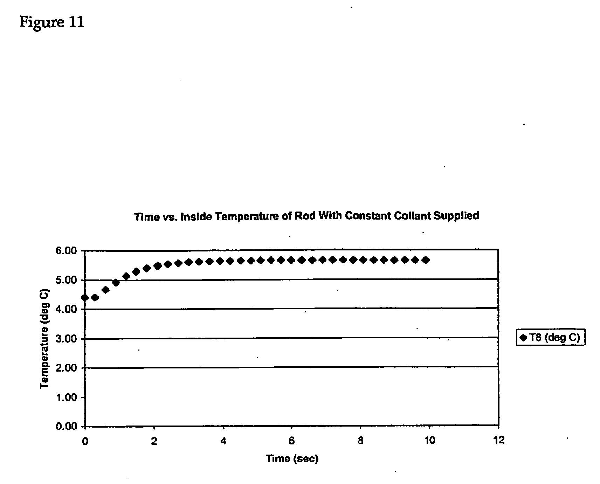

According to the present non-limiting example, the Nitinol nail and improved jig both have a 4 mm I.D., and the guide wire is around 2 mm in O.D. Therefore, there is an approximately 1 mm clearance space between rod and guide wire. The clearance space provides a path for the chilled saline to flow along. A tube may be fixed to the top of the improved jig to bring in the chilled saline during the procedure. Calculations demonstrate that a slow, steady flow of saline into the rod, creating a convection boundary, will allow the Nitinol device to reach an equilibrium temperature of about 5.65°C, which is below the austenite finishing temperature of the metal, thus allowing the rod to remain flexible (Figure 11).

The saline will be able to flow out of the bottom end of the rod and into the body via the fracture. Any excess saline will flow out of the top of the device. The chilled saline itself should be at a physiologically safe temperature, e.g., approximately 1-5°C. This is just above freezing, preventing the destruction of skin and muscle tissue, but cool enough to prevent the rod from completely reaching its austenite phase. A hole in the locking screw centering device, which protrudes from the improved jig (Figure 9) is used to align the locking screw into the proximal portion of the nail. When the nail is threaded onto the improved jig, the hole on the nail and the hole on the locking screw centering device should align. The distal end of the nail may be aligned and fixed with the aid of an x-ray or other method known in the art.

### Manufacturing

The mechanical properties of Nitinol may be significantly changed during the course of fabrication and machining. Due to the alloy's elasticity, high titanium content, and work-hardening rate, the alloy presents challenges in the production of a finished part. According to this example, which is not intended to be limiting, the Nitinol alloy comprises about 55.5 % nickel, 0.05% carbon, 0.005% hydrogen, 0.05% oxygen, 0.05% copper and the remaining (~44.345%) titanium. However, one skilled in the art may use another formulation in the practice of this invention, so long as the austenite start temperature is at a physiologically safe temperature, wherein physiologically safe temperature means a temperature that does not cause or facilitate permanent tissue damage over the time course required to deliver the Nitinol nail to the intramedullary canal.

During manufacturing, once the alloy is melted into its composition of Nickel and Titanium, it is usually forged and rolled into bar or slab form. Hot-working has been found to break down the cast structure and improve mechanical properties. Once hot-worked, the alloy is then cold-worked. The cold-working process can be challenging because of the alloy's work-hardening rate. Cold-working and heat-treating must be done to achieve final dimensions and desired physical and mechanical properties. The alloy is difficult to form at ambient temperatures due to Nitinol's super elastic properties and its tendency to return to its original shape once deformed. Also, when trying to heat treat a part made of Nitinol, the part should be fully constrained in the desired shape to prevent the part from trying to return to its original shape.

Once the metal has been formed and heat treated, the sample can be machined to its desired shape. Conventional techniques of milling, turning, and drilling can be used to machine Nitinol to its desired shape. Carbide tools with chlorinated lubricant are recommended for these operations.

In this particular example, gun drilling was chosen to drill the center of the Nitinol nail, mainly due to the fact that a normal drill bit may not be strong enough and may become brittle with the increasing heat from friction. Gun drilling includes three main components: a carbide tip, a heat treated alloy shank, and a steel driver. These components are hollow, allowing coolant to pass through the entire configuration. This coolant to keeps the drill bit from overheating during the cutting process. Once a hole has been started in the center of the tube, the drill is positioned and forced through the workpiece, creating thin curled chips of the NitinoL The coolant not only cools the tool, but also carries the chips away from the drill area.

After gun drilling, the rod would be finished on a lathe and tapered at the end. Also, the top of the rod may possess threads to accept the insertion device (jig). An end cap will be placed in this threaded hole after insertion in order to reduce the risk of bone in-growth. After manufacturing, the rod may be bent, placed in a mold and baked to achieve a slight bend at the top to allow the device to conform to the bone cavity and any angled hole that may be drilled into the bone. In this particular curved embodiment, markings may be made on the Nitinol nail to indicate the correct orientation of the device, such that the pre-bent portion of the rod coincides with the angled hole drilled into the bone cavity.

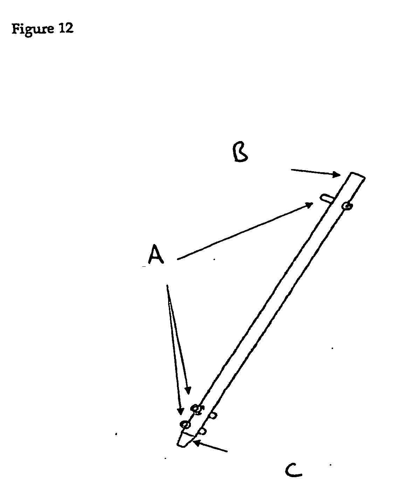

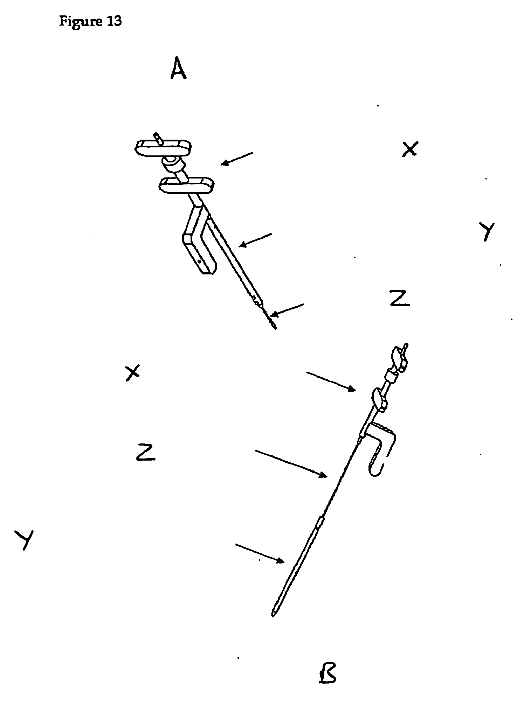

Figure 12 depicts a Nitinol nail in a straight conformation and with locking screws. Figure 13 depicts an assembly useful in the insertion of a Nitinol nail of the present invention. The assembly comprises an improved jig, a guide wire and an intramedullary nail.

### Example 3: Braided Nitinol nail

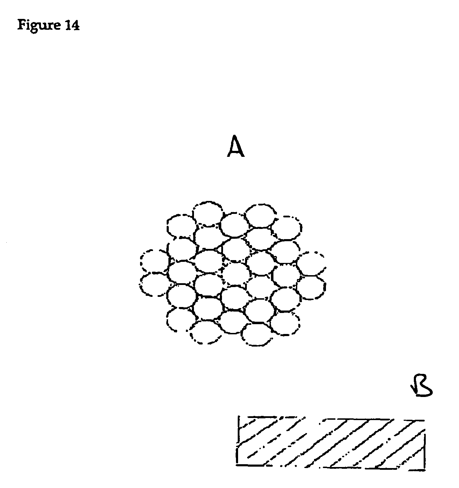

In another embodiment, the Nitinol intramedullary nail comprises a braided rod made of many small diameter cylinders composed of body temperature Nitinol (Figure 14). This embodiment includes parameters that are very similar to that of a single piece nail (supra, Example 2), as well as additional different parameters.

A particular advantage to a Nitinol nail comprising a multiplicity of small diameter cylinders is that the nail possibly may be easier to remove from the intramedullary canal. That is, chilled saline may be injected into the nail to allow the nail to enter the martensite state and become more malleable. Because the particular nail of this example is made up of many different cylinders, the chilled saline (chilled saline is at a temperature below the austenite start temperature) injected into the tubes making up the nail will come into contact with more surface area than a single hollow nail would. As the saline flows through the tubes, it cools the Nitinol. The mechanical properties will be altered to allow the surgeon to bend the device to the point where it can be removed from the bone.

In another aspect of this particular embodiment, the individual small diameter cylinders are fused together, preferably by soldering. Alternatively, a plastic sleeve may be molded around the braided nail to make an effectively single piece nail. An advantage to a plastic sleeve is that it can also serve as a barrier between the bone and rod to prevent in-growth of biological material into the nail. Plastics are available that have been used in prosthetics and are FDA approved (supra).

### Example 4: Surgical method

Prior to the surgery, the operating room should be supplied with all the necessary equipment for the surgery including the intramedullary nail, sterile chilled saline, and an appropriate sized guide wire. A section of a patient's limb is measured and a intramedullary nail of the proper size is selected. A nail insertion system, which comprises the Nitinol nail, a drill guide and a threaded jig (insertion device), is assembled. The nail insertion system is submerged in the sterile chilled saline at least about ten minutes prior to the surgery. The patient is positioned and immobilized as is appropriate for the limb or section of limb to which the surgery is to be performed.

In a situation is which the intramedullary nail is to be used in the repair of a humerus, the following procedures may be carried out However, the use of an intramedullary nail of the instant invention shall not be limited in scope to only repairing a humerus. While the following procedure describes the use of a Nitinol nail, the nail as described in Example 1 (e.g. link and wire system, supra) may also be used in this procedure.

The patient is positioned with his or her legs parallel to the floor and the upper body semi-reclined with the affected shoulder slightly over the edge of the operating table. The patient's head can be immobilized with one or two strips of adhesive tape to prevent movement while traction is being applied to the arm. A deltopectoral incision is made near the area of the humerus that is to be drilled. A deltoid splitting incision may used as an alternative with care given to avoid the axillary nerve. The superficial tissues are divided and the deltopectoral groove is blunt dissected to deepen the incision (use of a retractor may be needed to view the site to be drilled). A hole is drilled in the proximal region of the humerus large enough to fit the width of the Nitinol nail to be used (9-12 mm). Preferably, the hole should be drilled at an angle of between 30-35° pointing towards the distal end of the humerus relative to the central axis of the humerus. A bulb ended guide wire is inserted into the humeral canal. A flex reamer device having a diameter of preferably 0.5 mm larger than the diameter of the Nitinol nail is placed over the bulb ended guide wire and is used to clear away the soft tissue within the humeral intramedullary canal. The reamer is removed from the canal. A slipcover is placed over the bulb ended guide wire and inserted into the humeral intramedullary canal. The bulb ended guide wire is then removed from canal. A standard guide wire is then inserted through the slip cover and into the canal. The slip cover is removed from the canal in advance of the insertion of the Nitinol nail into the intramedullary canal.

After the canal has been reamed and guide wire inserted, the nail insertion system is removed from the chilled saline bath. The nail is fed into the insertion point and is hammered down the canal over the guide wire. The chilled saline is slowly injected to increase flexibility of the Nitinol nail. Once the nail is past the fracture point and almost completely in the humeral canal, the guide wire is removed.

Any necessary adjustments to the position of the nail are made before inserting any proximal and distal screws. The proximal end of the humerus is secured by inserting a titanium screw through a drill guide that is attached to the nail insertion system and screwing the screw through the humerus and into the predrilled holes in the nail. The hole in the distal end of the nail may be located using -ray techniques, a hole drilled through the distal part of the humerus aligned with the distal hole in the nail, and a screw is inserted through the distal humeral and nail holes to secure the distal end of the humerus. The jig, drill guide and threaded hammer cover are removed and disassembled after the rod is secured at the proximal and distal ends. The incision is then closed with an appropriate suture.

Table 1 illustrates how the Nitinol nail, compared to a standard titanium nail, can drastically reduce the time of surgery to fix a fractured humerus.

Table 1: Time Lapse: Original Humeral Procedure Compared to New Humeral Procedure.

| Time | Original Procedure | New Procedure |
|---|---|---|
| 15 min prior | Presurgical preparation | Assemble insertion device / Presurgical preparation |
| 10 min prior | | Submerge insertion device into ice saline bath |
| 5 min prior | Immobilize patient's head and shoulder | Immobilize patient's head and shoulder |
| 0 | Make incision at top of shoulder | Make a deltopectoral incision |
| 5 min | Divide superficial tissue | Divide superficial tissue |
| 10 min | Sever rotator cuff | Drill insertion hole into humerus |
| 15 min | | Ream inner humeral canal |
| 20 min | | Insert guidewire/nitinol rod |
| 25 min | | Remove guidewire/situate rod |
| 30 min | Drill insertion hole into top of humerus | Drill hole for proximal screw using drill guide / insert proximal screw |
| 35 min | | Drill hole for distal screw using x-ray / insert distal screw |
| 40 min | Ream inner humeral canal | |
| 45 min | Insert guidewire / nitinol rod | Dissassemble insertion apparatus |
| 50 min | | Suture incision |
| 55 min | Remove guidewire /situate rod | |
| 1hr 5min | Drill hole for proximal screw / insert proximal screw | |
| 1hr 15min | Drill hole for distal screw using x-ray / insert distal screw | |
| 1hr 35 min | Reatlach rotator cuff | |
| 2hr | suture incision | |

## Claims

1. An elongated cylindrical intramedullary nail, for providing support in a canal for which it is shaped to be inserted, having a lengthwise cylindrical axis extending from a proximal end to a distal end and having metal composition, which has a martensite state whereby the intramedullary nail can be bent to assume a desired shape to accommodate the insertion of the nail into an incision that is not aligned in-line to the intramedullary canal and, which has an austenite state whereby the intramedullary nail transitions to an effective shape, which shape corresponds to the originally manufactured shape and conforms to the intramedullary canal, and which nail is capable of transitioning from a flexible state in the martensite state to a rigid state in the austenite state and where said metal composition comprises a biocompatible material.

2. The intramedullary nail of Claim 1, wherein said metal composition comprises a nickel titanium alloy.

3. The intramedullary nail of Claim 2, wherein said metal composition comprises an alloy that consists of about 55.5 % nickel, about 0.05% carbon, about 0.005% hydrogen, 0.05% oxygen, 0.05% copper and about 44.345% titanium.

4. The intramedullary nail of Claim 3, wherein said metal composition comprises a body temperature Nitinol alloy.

5. The intramedullary nail of Claim 4, wherein said body temperature Nitinol Alloy comprises a nickel titanium alloy having an auslenile start temperature of 15°C ± 5°C and an austenite finish temperature of 35°C ± 5°C.

6. The intramedullary nail of Claim 5, wherein the intramedullary nail in the flexible state has a yield strength of 137.9 MPa ±34.47MPa (20 ksi ± 5 ksi).

7. The intramedullary nail of any one of Claims 1 to 6, wherein the intramedullary nail is suited for the repair of a fractured humerus.

8. The intramedullary nail of any preceding claim, wherein the intramedullary nail has a length of 250 mm ± 10% and an outer diameter tapering off from about 10 mm ± 10% to 9 mm ± 10% from proximal to distal end respectively.

9. The intramedullary nail of any preceding claim, wherein the intramedullary nail comprises multiple small cylinders braided together, wherein the small cylinders each comprise a nickel titanium alloy.

10. The intramedullary nail of claim 9 comprising a sleeve which is made of a biocompatible plastic material and which encases the multiple small cylinders.

11. The intramedullary nail of Claim 8, wherein the intramedullary nail is hollow and has an inner diameter of 4 mm ± 10%.

12. The intramedullary nail of any one of claims 1 to 10, wherein the bend off said cylindrical axis of said intramedullary nail in the rigid state is greater than 0° and less than or equal to 90° relative to the cylindrical axis when shaped for insertion in the canal, such that the bend accommodates an off-center point of insertion near said proximal end.

## Patentansprüche

1. Ein länglicher zylindrischer Marknagel zum Bereitstellen einer Stütze in einem Kanal, für den er zum Einführen vorgeformt ist, mit einer sich von einem proximalen Ende zu einem distalen Ende erstreckenden, längsgerichteten, zylindrischen Achse und einer Metallzusammensetzung, die einen martensitischen Zustand aufweist, wobei der Marknagel gebogen werden kann, um eine gewünschte Form anzunehmen, um die Einführung des Nagels in einen Einschnitt vorzunehmen, der nicht in einer Linie mit dem Markkanal ausgerichtet ist, und die einen austenitischen Zustand aufweist, wobei der Marknagel in eine wirksame Form übergeht, wobei diese Form mit der ursprünglich hergestellten Form übereinstimmt und dem Markkanal entspricht, und wobei der Nagel dazu geeignet ist, von einem flexiblen Zustand in dem martensitischen Zustand zu einem steifen Zustand in dem austenitischen Zustand überzugehen, und wobei die Metallzusammensetzung ein bioverträgliches Material aufweist.

2. Der Marknagel gemäß Anspruch 1, wobei die Metallzusammensetzung eine Nickel-Titan-Legierung aufweist.

3. Der Marknagel gemäß Anspruch 2, wobei die Metallzusammensetzung eine Legierung aufweist, die aus ungefähr 55,5 % Nickel, ungefähr 0,05 % Kohlenstoff, ungefähr 0,005 % Wasserstoff, 0,05 % Sauerstoff, 0,05 % Kupfer und ungefähr 44,345 % Titan besteht.

4. Der Marknagel gemäß Anspruch 3, wobei die Metallzusammensetzung eine Körpertemperatur-Nitinol-Legierung aufweist.

5. Der Marknagel gemäß Anspruch 4, wobei die Körpertemperatur-Nitinol-Legierung eine Nickel-Titan-Legierung mit einer Austenitstarttemperatur von 15 °C ± 5 °C und einer Austenitendtemperatur von 35 °C ± 5 °C aufweist.

6. Der Marknagel gemäß Anspruch 5, wobei der Marknagel in dem flexiblen Zustand eine Fließfestigkeit von 137,9 MPa ± 34,47 MPa (20 ksi ± 5 ksi) aufweist.

7. Der Marknagel gemäß einem der Ansprüche 1 bis 6, wobei der Marknagel zur Reparatur von gebrochenen Oberarmknochen geeignet ist.

8. Der Marknagel gemäß einem der vorhergehenden Ansprüche, wobei der Marknagel eine Länge von 250 mm ± 10 % aufweist und einen äußeren Durchmesser aufweist, der sich von circa 10 mm ± 10 % zu 9 mm ± 10 % von dem proximalen zu dem entsprechenden distalen Ende verjüngt.

9. Der Marknagel gemäß einem der vorhergehenden Ansprüche, wobei der Marknagel mehrere kleine miteinander verflochtene Zylinder aufweist, wobei die kleinen Zylinder jeweils eine Nickel-Titan-Legierung aufweisen.

10. Der Marknagel gemäß Anspruch 9 mit einer Hülle, welche aus einem biokompatiblen Kunststoffmaterial besteht und welche die mehreren kleinen Zylinder umhüllt.

11. Der Marknagel gemäß Anspruch 8, wobei der Marknagel hohl ist und einen inneren Durchmesser von 4 mm ± 10 % aufweist.

12. Der Marknagel gemäß einem der Ansprüche 1 bis 10, wobei die Krümmung der zylindrischen Achse des Marknagels in dem steifen Zustand größer als 0° und kleiner oder gleich 90° relativ zu der zylindrischen Achse ist, wenn er zur Einführung in den Kanal geformt ist, so dass die Krümmung einen außermittigen Einführungspunkt nahe des proximalen Endes aufnimmt.

## Revendications

1. Clou intramédullaire cylindrique allongé, pour fournir un support dans un canal pour lequel il est formé afin d'être inséré, ayant un axe cylindrique dans le sens de la longueur s'étendant d'une extrémité proximale à une extrémité distale et ayant une composition de métal, qui a un état martensitique moyennant quoi le clou intramédullaire peut être fléchi pour adopter une forme souhaitée pour permettre l'insertion du clou dans une incision qui n'est pas alignée en ligne avec le canal intramédullaire et qui a un état austénitique moyennant quoi le clou intramédullaire passe à une forme effective, laquelle forme correspond à la forme fabriquée à l'origine et se conforme au canal intramédullaire, et lequel clou est capable de passer d'un état flexible dans l'état martensitique à un état rigide dans l'état austénitique et où ladite composition de métal comprend un matériau biocompatible.

2. Clou intramédullaire selon la revendication 1, dans lequel ladite composition de métal comprend un alliage de nickel-titane.

3. Clou intramédullaire selon la revendication 2, dans lequel ladite composition de métal comprend un alliage qui se compose d'environ 55,5% de nickel, environ 0,05% de carbone, environ 0,005% d'hydrogène, 0,05% d'oxygène, 0,05% de cuivre et environ 44,345% de titane.

4. Clou intramédullaire selon la revendication 3, dans lequel ladite composition de métal comprend un alliage de Nitinol à la température corporelle.

5. Clou intramédullaire selon la revendication 4, dans lequel ledit alliage de Nitinol à température corporelle comprend un alliage de nickel-titane ayant une température de début d'austénite de 15°C ± 5°C et une température de fin d'austénite de 35°C ± 5°C.

6. Clou intramédullaire selon la revendication 5, dans lequel le clou intramédullaire dans l'état flexible a une limite d'élasticité de 137,9 MPa ± 34,47 MPa (20 ksi ± 5 ksi).

7. Clou intramédullaire selon l'une quelconque des revendications 1 à 6, dans lequel le clou intramédullaire est adapté à la réparation d'un humérus fracturé.

8. Clou intramédullaire selon l'une quelconque des revendications précédentes, dans lequel le clou intramédullaire a une longueur de 250 mm ± 10% et un diamètre externe s'effilant d'environ 10 mm ± 10% à 9 mm ± 10% de l'extrémité proximale à l'extrémité distale respectivement.

9. Clou intramédullaire selon l'une quelconque des revendications précédentes, dans lequel le clou intramédullaire comprend de multiples petits cylindres tressés ensemble, où les petits cylindres comprennent chacun un alliage de nickel-titane.

10. Clou intramédullaire selon la revendication 9, comprenant un manchon qui est constitué d'un matériau plastique biocompatible et qui loge les multiples petits cylindres.

11. Clou intramédullaire selon la revendication 8, dans lequel le clou intramédullaire est creux et a un diamètre interne de 4 mm ± 10%.

12. Clou intramédullaire selon l'une quelconque des revendications 1 à 10, dans lequel le coude relatif audit axe cylindrique dudit clou intramédullaire dans l'état rigide est supérieur à 0° et inférieur ou égal à 90° par rapport à l'axe cylindrique lorsqu'il est formé pour insertion dans le canal, de telle sorte que le coude accueille un point d'insertion excentré à proximité de ladite extrémité proximale.
